# EUROPÄISCHE PATENTANMELDUNG

(11) **EP 1 247 799 A2**
(43) Veröffentlichungstag der Anmeldung: **09.10.2002**
(21) Anmeldenummer: 02007283.1
(22) Anmeldetag: 02.04.2002
(51) Int. Cl.: C07C 209/26

(54) **Verfahren zur Herstellung von N-Benzylaminen**

(30) Priorität: 04.04.2001 DE 10116816
(71) Anmelder: BASF AKTIENGESELLSCHAFT, 67056 Ludwigshafen (DE)
(72) Erfinder: Winsel, Harald, Dr., 67134 Birkenheide (DE); Siegel, Wolfgang, Dr., 67117 Limburgerhof (DE); Bartsch, Michael, Dr., 67433 Neustadt (DE)
(74) Vertreter: Isenbruck, Günter, Dr.

(57) **Zusammenfassung**

Verfahren zur Herstellung von N-Benzylaminen, bei dem
(i) in einem ersten Schritt ein Benzaldehyd mit einem primären Amin zum Imin umgesetzt wird, und
(ii) in einem zweiten Schritt das Imin mit Wasserstoff in Gegenwart eines Katalysators, der ein oder mehrere Metalle der Gruppen 8 bis 10 des Periodensystems enthält, zum N-Benzylamin hydriert wird,
wobei die Iminierung (i) in einem mit Wasser mischbaren Lösungsmittel durchgeführt und das entstehende Reaktionswasser nicht entfernt wird, und die Hydrierung (ii) in der bei der Iminierung (i) erhaltenen, Reaktionswasser enthaltenden Lösung des Imins durchgeführt wird.

## Beschreibung

Die Erfindung betrifft ein Verfahren zur Herstellung von N-Benzylaminen.

Es ist bekannt, N-Benzyl-α-methylbenzylamin durch Iminierung von Benzaldehyd mit α-Methylbenzylamin und nachfolgender Hydrierung des gebildeten Imins herzustellen. Ein derartiges Verfahren beschreiben Huckabee et al., Organic Process Research & Development 2000, 4, 594 - 595. Dabei werden optisch reines (R)- bzw. (S)-N-Benzyl-α-methylbenzylamin aus Benzaldehyd und (R)- bzw. (S)-α-Phenylethylamin über die palladiumkatalysierte Hydrierung von als Zwischenprodukt gebildetem Benzyliden-(1-phenylethyl)imin erhalten. Die Iminierung wird in Toluol als Lösungsmittel durchgeführt und das gebildete Reaktionswasser durch azeotrope Destillation entfernt.

Aufgabe der Erfindung ist es, ein einfacheres Verfahren zur Herstellung von N-Benzylaminen bereitzustellen.

Gelöst wird die Aufgabe durch ein Verfahren zur Herstellung von N-Benzylaminen, bei dem
(i) in einem ersten Schritt ein Benzaldehyd mit einem primären Amin zum Imin umgesetzt wird, und
(ii) in einem zweiten Schritt das Imin mit Wasserstoff in Gegenwart eines Katalysators, der ein oder mehrere Metalle der Gruppen 8 bis 10 des Periodensystems enthält, zum N-Benzylamin hydriert wird,
das dadurch gekennzeichnet ist, daß die Iminierung (i) in einem mit Wasser mischbaren Lösungsmittel durchgeführt und das entstehende Reaktionswasser nicht entfernt wird, und die Hydrierung (ii) in der bei der Iminierung (i) erhaltenen, Reaktionswasser enthaltenden Lösung des Imins durchgeführt wird.

Bevorzugte, nach dem erfindungsgemäßen Verfahren hergestellte N-Benzylamine sind solche der allgemeinen Formel (I) 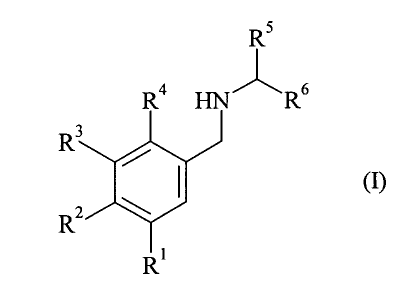 worin
- R¹, R², R³ und R⁴: unabhängig voneinander H, Halogen und gegebenenfalls mit Halogen ein- oder mehrfach substituiertes C₁-C₄-Alkyl oder-Alkoxy,
- R⁵: H oder C₁-C₈-Alkyl, und
- R⁶: H, C₁-C₈-Alkyl oder C₅-C₁₂-Aryl oder -Cycloalkyl, das mit Halogen, OH und gegebenenfalls mit Halogen ein- oder mehrfach substituiertem C₁-C₄-Alkyl- und/oder -Alkoxy ein- oder mehrfach substituiert sein kann, sowie mit C₁-C₄-Alkyloxy oder Benzyloxy substituiertes C₁-C₄-Alkyl,
bedeuten.

Diese werden erhalten, indem
(i) in einem ersten Schritt ein Benzaldehyd der allgemeinen Formel (II) mit einem Amin der allgemeinen Formel (III) 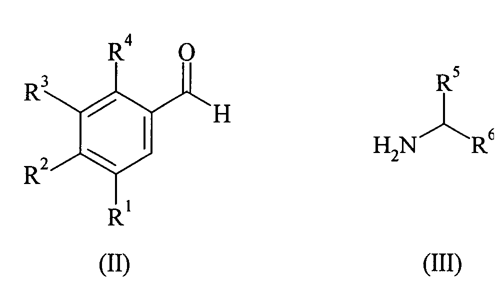 zum Imin der allgemeinen Formel (IV) umgesetzt wird, 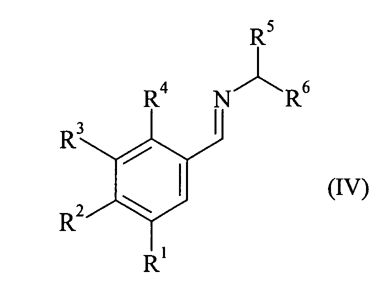 worin
   R¹ - R⁶ die oben angegebenen Bedeutungen haben, und
(ii) in einem zweiten Schritt das Imin der allgemeinen Formel (IV) mit Wasserstoff in Gegenwart eines Katalysators, der ein oder mehrere Metalle der Gruppen 8 bis 10 des Periodensystems enthält, zum N-Benzylamin der allgemeinen Formel (I) hydriert wird,
wobei die Iminierung (i) in einem mit Wasser mischbaren Lösungsmittel durchgeführt und das entstehende Reaktionswasser nicht entfernt wird, und die Hydrierung (ii) in der bei der Iminierung (i) erhaltenen, Reaktionswasser enthaltenden Lösung des Imins (IV) durchgeführt wird.

In einem ersten Schritt (i) wird ein Benzaldehyd, bevorzugt ein Benzaldehyd der allgemeinen Formel (II), mit einem Amin, bevorzugt einem Amin der allgemeinen Formel (III), zum einem Imin, bevorzugt der allgemeinen Formel (IV), umgesetzt. Reste R¹ bis R⁴ sind beispielsweise Fluor, Chlor, Brom, Iod, Methyl, Trifluormethyl, Ethyl, n-Propyl, i-Propyl, n-Butyl, i-Butyl, sec-Butyl, tert.-Butyl und die entsprechenden Alkoxyreste. Die Benzaldehyde (II) können bis zu vierfach substituiert sein, bevorzugt sind sie unsubstituiert oder ein- oder zweifach substituiert. Benzaldehyde der allgemeinen Formel (II) sind beispielsweise Benzaldehyd, 4-Methoxybenzaldehyd, 4-t-Butylbenzaldehyd, 4-Methylbenzaldehyd, 4-Chlorbenzaldehyd, 4-Fluorbenzaldehyd, 3-Fluorbenzaldehyd, 3,5-Difluorbenzaldehyd, 4-Trifluormethylbenzaldehyd und 3,5-Bistrifluormethylbenzaldehyd.

Reste R⁵ und R⁶ sind beispielsweise Methyl, Ethyl, Propyl, i-Propyl, 1- oder 2-Butyl, 1-, 2 - oder 3-Pentyl, 1-,2- oder 3-Hexyl, 1-, 2-, 3- oder 4-Heptyl und 1-, 2-, 3- oder 4-Octyl. R⁶ kann ferner unsubstituiertes oder mit OH, den genannten Halogenen, den genannten gegebenenfalls substituierten C₁-C₄-Alkyl- und -Alkoxyresten ein oder mehrfach substituiertes, bevorzugt ein- oder zweifach substituiertes C₅-C₁₂-Aryl oder -Cycloalkyl sein, wie unsubstituiertes oder derart substituiertes Phenyl, Naphthyl, Tetrahydronaphthyl, Indyl, Cyclopentyl oder Cyclohexyl. Amine der allgemeinen Formel (III) sind beispielsweise α-Phenylethylamin, 1-(4-Methylphenyl)ethylamin, 2-Heptylamin, 1-(4-Chlorphenyl)ethylamin, 1-Phenylpropylamin, 2-Amino-3,3-dimethylbutan, 1-(1-Naphthyl)ethylamin, 1,2,3,4-Tetrahydronaphthylamin, 1-(4-Methoxyphenyl)ethylamin, Aminocyclopentanol, Aminocyclohexanol, 1-Aminoindan, 1-(2,4-Dichlorphenyl)ethylamin, 1-Benzyloxy-2-aminopropan, 1-Benzyloxy-3-aminobutan, 1-(2-Naphthyl)ethylamin, 1-(3,5-Bis-trifluormethylphenyl)ethylamin und 1-(4-Fluorphenyl)ethylamin.

Die Iminierung (i) wird in homogener Phase in einem mit Wasser mischbaren Lösungsmittel durchgeführt. Geeignete mit Wasser mischbare Lösungsmittel sind Methanol, Ethanol, n-Propanol, iso-Propanol, Glykol, t-Butanol, THF, Glykolmono- oder - dialkylether wie Dimethoxyethan. Bevorzugte Lösungsmittel sind Methanol, Ethanol, n-Propanol und iso-Propanol, besonders bevorzugtes Lösungsmittel ist Methanol. Üblicherweise beträgt die Konzentration des Benzaldehyds und die des Amins zusammen von 10 bis 65 Gew.-%.

Die Iminierung (i) wird vorzugsweise bei einer Temperatur von 10 bis 40°C, besonders bevorzugt bei 20 bis 30°C und bevorzugt bei Atmosphärendruck durchgeführt. Üblicherweise ist eine Reaktionszeit von 0,1 bis 5 h für die Iminierung (i) ausreichend.

Im Anschluß an die Iminierung wird ohne vorherige Abtrennung des Reaktionswassers das gebildete Imin mit Wasserstoff in Gegenwart eines Palladium enthaltenden Katalysators in der das Reaktionswasser enthaltenden Lösung des Imins hydriert. Geeignete Katalysatoren enthalten ein oder mehrere Metalle der Gruppen 8 bis 10 des Periodensystems auf einem beliebigen üblichen anorganischen Träger wie Aluminiumoxid, Siliciumdioxid, Zirkondioxid, Titandioxid oder Kohlenstoff. Bevorzugte Katalysatoren enthalten Platin, Palladium, Nickel oder Rhodium auf einem anorganischen Träger, besonders bevorzugt ist Palladium auf Aktivkohle.

Die Hydrierung (ii) wird vorzugsweise bei einer Temperatur von 10 bis 40°C, besonders bevorzugt 20 bis 30°C durchgeführt, wobei der Wasserstoffdruck üblicherweise von 0,1 bis 25 bar, vorzugsweise von 0,1 bis 5 bar beträgt. Üblicherweise ist eine Reaktionszeit von 1 bis 100 h für die Hydrierung ausreichend.

Der Hydrierungskatalysator kann vor oder nach der Iminierung (i) zugesetzt werden. Das Verfahren kann kontinuierlich oder diskontinuierlich betrieben werden und verläuft bei Einsatz von chiralen Aminen ohne Racemisierung.

Durch den Wegfall der azeotropen Destillation wird eine thermische Beanspruchung des als Zwischenprodukt gebildeten Imins vermieden. Ferner ist das erfindungsgemäße Verfahren gegenüber dem aus dem Stand der Technik bekannten Verfahren einfacher durchzuführen, da die azeotrope Destillation als Verfahrensoperation entfällt.

Die Erfindung wird durch die nachstehenden Beispiele näher erläutert.

### Beispiele

### Beispiel 1: diskontinuierliche Synthese von N-4-Methoxybenzyl-1-phenylethylamin

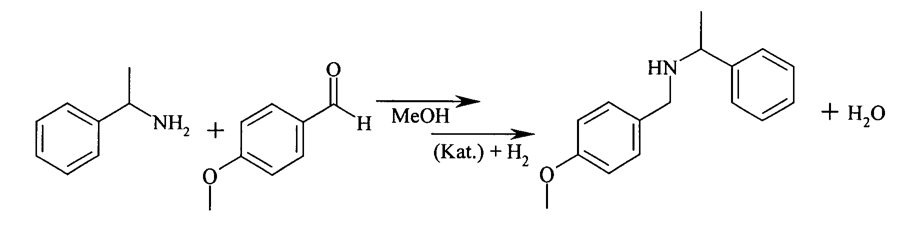

100 ml Methanol und 100 mmol 1-Phenylethylamin werden vorgelegt und 100 mmol 4-Methoxybenzaldehyd bei 24°C innerhalb von 15 min zugetropft. Man rührt 6 h bei 24°C nach, kontrolliert auf Vollständigkeit der Iminierung per GC, gibt 0,5 g Pd/C (10 Gew.-%) zu und hydriert das Rohgemisch mit Wasserstoff 5 h bei Atmosphärendruck. Man entnimmt eine Probe und untersucht die Zusammensetzung per GC-Analyse.

| GC-Analyseergebnisse (in GC-Flächenprozent): | |
|---|---|
| 1-Phenylethylamin | 4,7 % |
| 4-Methoxybenzaldehyd | 0,2 % |
| N-4-Methoxybenzyl-1-phenylethylamin | 89,5 % |

### Beispiel 2: diskontinuierliche Synthese von N-Benzyl-1-(4-methylphenyl)ethylamin

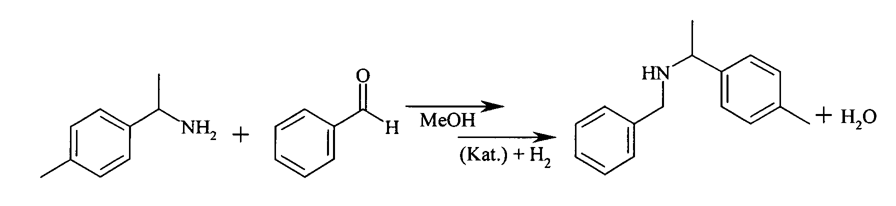

100 ml Methanol und 100 mmol 4-Methyl-1-phenylethylamin werden vorgelegt und 100 mmol Benzaldehyd bei 24°C innerhalb von 15 min zugetropft. Man rührt 6 h bei 24°C nach, kontrolliert auf Vollständigkeit der Iminierung per GC, gibt 0,5 g Pd/C (10 Gew.-%) zu und hydriert das Rohgemisch mit Wasserstoff 5 h bei Atmosphärendruck. Man entnimmt eine Probe und untersucht die Zusammensetzung per GC-Analyse.

| GC-Analyseergebnisse (in GC-Flächenprozent): | |
|---|---|
| 4-Methyl-1-phenylethylamin | 5,1 % |
| Benzaldehyd | 1,3 % |
| N-Benzyl-1-(4-methylphenyl)ethylamin | 82,5 % |

### Beispiel 3: diskontinuierliche Synthese von (R)-N-Benzyl-1-phenylethylamin

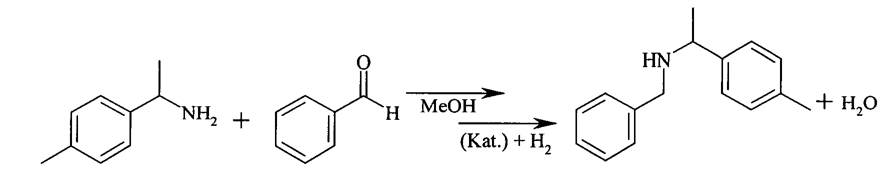

412 kg Methanol und 375,7 kg (R)-1-Phenylethylamin (ee = 97,1 %) werden vorgelegt und 329 kg Benzaldehyd bei 24°C innerhalb von 120 min zugetropft. Man rührt 6 h bei 24°C nach, kontrolliert auf Vollständigkeit der Iminierung per GC, gibt 6 kg Pd/C (5 Gew.- %) zu und hydriert das Rohgemisch mit Wasserstoff 24 h bei Atmosphärendruck. Man entnimmt eine Probe und untersucht die Zusammensetzung per GC-Analyse.

| GC-Analyseergebnisse (in GC-Flächenprozent): | |
|---|---|
| (R)-1-Phenylethylamin | 0 % |
| Benzaldehyd | 0,1 % |
| (R)-N-Benzyl-1-phenylethylamin ee = 97,1 % | 99,4 % |

### Beispiel 4: diskontinuierliche Synthese von N-Benzyl-2-methylhexylamin

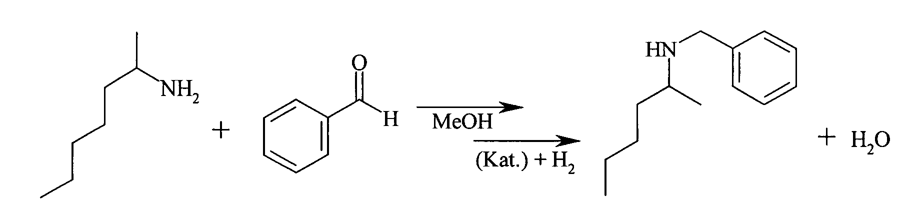

100 ml Methanol und 100 mmol 2-Heptylamin werden vorgelegt und 100 mmol Benzaldehyd bei 24°C innerhalb von 15 min zugetropft. Man rührt 6 h bei 24°C nach, kontrolliert auf Vollständigkeit der Iminierung per GC, gibt 0,5 g Pd/C (10 Gew.-%) zu und hydriert das Rohgemisch mit Wasserstoff 5 h bei Atmosphärendruck. Man entnimmt eine Probe und untersucht die Zusammensetzung per GC-Analyse.

| GC-Analyseergebnisse (in GC-Flächenprozent): | |
|---|---|
| 2-Heptylamin | 0,6 % |
| Benzaldehyd | 0,0 % |
| N-Benzyl-2-methylhexylamin | 97,8 % |

### Beispiel 5: diskontinuierliche Synthese von N-Benzyl-1-naphthylethylamin

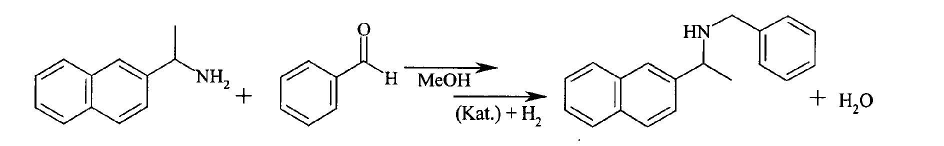

100 ml Methanol und 350 mmol 1-Naphthylethylamin werden vorgelegt und 350 mmol Benzaldehyd bei 24°C innerhalb von 15 min zugetropft. Man rührt 6 h bei 24°C nach, kontrolliert auf Vollständigkeit der Iminierung per GC, gibt 0,5 g Pd/C (10 Gew.-%) zu und hydriert das Rohgemisch mit Wasserstoff 5 h bei Atmosphärendruck. Man entnimmt eine Probe und untersucht die Zusammensetzung per GC-Analyse.

| GC-Analyseergebnisse (in GC-Flächenprozent): | |
|---|---|
| 1-Naphthylethylamin | 0,6 % |
| Benzaldehyd | 0,0 % |
| N-Benzyl-1-naphthylethylamin | 94,3 % |

### Beispiel 6: kontinuierliche Synthese von (S)-N-Benzyl-1-phenylethylamin

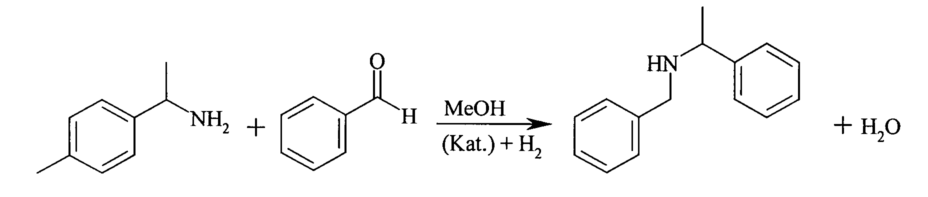

1185 g Methanol, 605,9 g (S)-Phenylethylamin (ee = 99,2 %) und 530,6 g Benzaldehyd werden bei Raumtemperatur gemischt und das entstandene Imin nach 30 min in einem Festbett-Rohrreaktor bei Raumtemperatur und 10 bar Wasserstoffdruck an 144 g eines palladiumhaltigen Hydrierkatalysators (1 Gew.- % Pd, 10 Gew.-% MgO auf Al₂O₃) zum (S)-N-Benzylphenylethylamin hydriert. Man entnimmt am Reaktorausgang eine Probe und untersucht die Zusammensetzung per GC-Analyse. Die Aufarbeitung erfolgt destillativ.

| GC-Analyseergebnisse (in GC-Flächenprozent): | |
|---|---|
| (S)-Phenylethylamin | 0,1 % |
| Benzaldehyd | 0 % |
| (S)-N-Benzyl-1-phenylethylamin ee=99,1 % | 98,3 % |

## Patentansprüche

1. Verfahren zur Herstellung von N-Benzylaminen, bei dem
(i) in einem ersten Schritt ein Benzaldehyd mit einem primären Amin zum Imin umgesetzt wird, und
(ii) in einem zweiten Schritt das Imin mit Wasserstoff in Gegenwart eines Katalysators, der ein oder mehrere Metalle der Gruppen 8 bis 10 des Periodensystems enthält, zum N-Benzylamin hydriert wird,
**dadurch gekennzeichnet, daß** die Iminierung (i) in einem mit Wasser mischbaren Lösungsmittel durchgeführt und das entstehende Reaktionswasser nicht entfernt wird, und die Hydrierung (ii) in der bei der Iminierung (i) erhaltenen, Reaktionswasser enthaltenden Lösung des Imins durchgeführt wird.

2. Verfahren zur Herstellung von N-Benzylaminen der allgemeinen Formel (I) 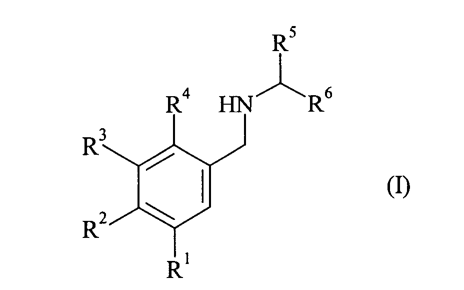 worin
R¹, R², R³ und R⁴ unabhängig voneinander H, Halogen oder gegebenenfalls mit Halogen ein oder mehrfach substituiertes C₁-C₄-Alkyl oder-Alkoxy,
R⁵ H oder C₁-C₈-Alkyl, und
R⁶ H, C₁-C₈-Alkyl oder C₅-C₁₂-Aryl oder -Cycloalkyl, das mit Halogen, OH und gegebenenfalls mit Halogen ein- oder mehrfach substituiertem C₁-C₄-Alkyl- und/oder -Alkoxy ein - oder mehrfach substituiert sein kann, sowie mit C₁-C₄-Alkyloxy oder Benzyloxy substituiertes C₁-C₄-Alkyl,
bedeuten,
wobei
(i) in einem ersten Schritt ein Benzaldehyd der allgemeinen Formel (II) mit einem Amin der allgemeinen Formel (III) 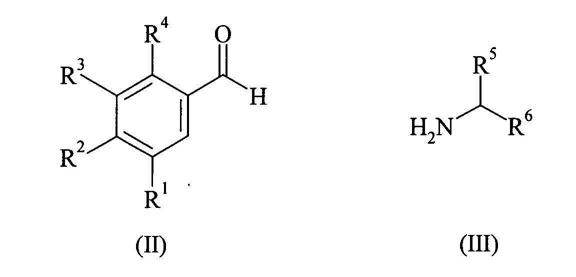 zum Imin der allgemeinen Formel (IV) umgesetzt wird, 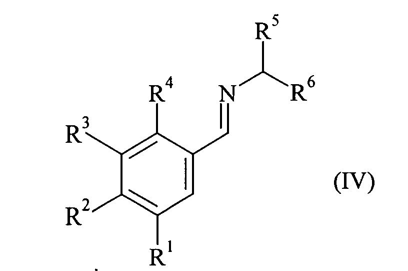 worin
R¹ - R⁶ die oben angegebene Bedeutung haben, und
(ii) in einem zweiten Schritt das Imin der allgemeinen Formel (IV) mit Wasserstoff in Gegenwart eines Katalysators, der ein oder mehrere Metalle der Gruppen 8 bis 10 des Periodensystems enthält, zum N-Benzylamin der allgemeinen Formel (I) hydriert wird,
**dadurch gekennzeichnet, daß** die Iminierung (i) in einem mit Wasser mischbaren Lösungsmittel durchgeführt und das entstehende Reaktionswasser nicht entfernt wird, und die Hydrierung (ii) in der bei der Iminierung (i) erhaltenen, Reaktionswasser enthaltenden Lösung des Imins (IV) durchgeführt wird.

3. Verfahren nach Anspruch 1 oder 2, **dadurch gekennzeichnet, daß** das mit Wasser mischbare Lösungsmittel ausgewählt ist aus der Gruppe bestehend aus Methanol, Ethanol, n-Propanol, iso-Propanol, Glykol, t-Butanol, THF, und Glykolmono- und -dialkylethern.

4. Verfahren nach einem der Ansprüche 1 bis 3, **dadurch gekennzeichnet, daß** als Katalysator Palladium auf Aktivkohle eingesetzt wird.

5. Verfahren nach einem der Ansprüche 2 bis 4, **dadurch gekennzeichnet, daß** der Aldehyd der allgemeinen Formel (II) ausgewählt ist aus der Gruppe bestehend aus Benzaldehyd, 4-Methoxybenzaldehyd, 4-t-Butylbenzaldehyd, 4-Methylbenzaldehyd, 4-Chlorbenzaldehyd, 4-Fluorbenzaldehyd, 3-Fluorbenzaldehyd, 3,5-Difluorbenzaldehyd, 4-Trifluormethylbenzaldehyd und 3,5-Bistrifluormethylbenzaldehyd.

6. Verfahren nach einem der Ansprüche 2 bis 5, **dadurch gekennzeichnet, daß** das Amin der allgemeinen Formel (III) ausgewählt ist aus der Gruppe bestehend aus α-Phenylethylamin, 1-(4-Methylphenyl)ethylamin, 2-Heptylamin, 1-(4-Chlorphenyl)ethylamin, 1-Phenylpropylamin, 2-Amino-3,3-dimethylbutan, 1-(1-Naphthyl)ethylamin, 1,2,3,4-Tetrahydronaphthylamin, 1-(4-Methoxyphenyl)ethylamin, Aminocyclopentanol, Aminocyclohexanol, 1-Aminoindan, 1-(2,4-Dichlorphenyl)ethylamin, 1-Benzyloxy-2-propylamin, 1-(2-Naphthyl)ethylamin, 1-(3,5-Bis-trifluormethylphenyl)ethylamin und 1-(4-Fluorphenyl)ethylamin.

7. Verfahren nach einem der Ansprüche 1 bis 6, **dadurch gekennzeichnet, daß** die Iminierung (i) bei einer Temperatur von 10 bis 40°C und bei Atmosphärendruck durchgeführt wird.
